# EUROPEAN PATENT APPLICATION

(11) **EP 4 728 996 A1**
(43) Date of publication of application: **22.04.2026**
(21) Application number: 24206664.5
(22) Date of filing: 15.10.2024
(51) Int. Cl.: A61B 10/02, A61B 17/3205, A61B 10/06

(54) **SKIN PUNCH BIOPSY TOOL**

(71) Applicant: Smart Punch ApS, 1420 København K (DK)
(72) Inventor: Gyrn, Steffen, 1420 København K (DK); LeFévre, Anne Sofie, 1420 København K (DK); PALLESEN, Kristine Appel, 5230 Odense M (DK)
(74) Representative: Larsen & Birkeholm A/S

(57) **Abstract**

The present invention relates to a combination skin punch biopsy tool comprising both skin cutting means creating a skin punch biopsy from mucous membrane or skin, and a gripping part for catching and safe-handling of the mucous membrane or skin sample.

In particular, the present invention relates to a skin punch biopsy tool for providing a skin or mucous membrane sample (15) comprising primary cutting means in form of a cutting edge (1) and gripping means (2, 2a, 2b) for gripping a skin sample (15), wherein the cutting edge(s) (1) is not part of the gripping means (2, 2a, 2b). The primary cutting edge(s) 1 is/are separate from the gripping means and normally positioned at a different position than the gripping means 2, 2a, 2b and constituted of parts being completely or at least partly separated from the gripping means 2 i.e. the primary cutting means 1 are not constituted of the same part(s) as the gripping means 2.

## Description

The present invention relates to a combination skin punch biopsy tool comprising both skin cutting means creating a skin punch biopsy from mucous membrane or skin, and a gripping part for catching and safe-handling of the mucous membrane or skin sample.

In particular, the present invention relates to a skin punch biopsy tool comprising both a cutting edge and gripping means for gripping a sample which offers optimization of both parts. Also, the gripping means may comprise a secondary cutting edge replacing a separate scissor or knife for cutting the sample loose, i.e. a biopsy tool according to the invention may comprise a two-in-one tool or a three-in-one tool.

### Background of the invention

Today a normal way of obtaining a skin punch biopsy is to apply a series of tools, a punching device comprising a punching part provided with a cutting edge normally creating a cylindrical biopsy in an angle perpendicular to the skin surface, a gripping device such as a pair of tweezers gripping the biopsy after having removed the punching device, and a cutting tool which is used to free the biopsy at the bottom while holding the biopsy with the gripping device, which cutting tool may be a pair of scissors.

All tools used to prepare a biopsy are either discarded after use or are cleaned and autoclaved. When using two or three tools made primarily of stainless steel, both procedures are very expensive and environmental unfriendly, especially in the scenarios were all the tools are discarded. Another issue with the current punch biopsy procedures is that the punch diameters may vary from 2-12 mm and there are no gripping devices that match those different diameters. A standard tweezer is used for all sample sizes, which makes the sampling of the tissue challenged in several situations depending on the tissue viscosity. In some cases, the sample may break because of a bad fit between the size of the gripping device and the tissue and in other cases the sample may become damaged because it is being squeezed in the gripping, which damages the tissue and thus gives a more unprecise diagnostic.

It is known to combine a skin cutter with a gripping device. US 52009755 A discloses a skin cutter instrument for excising ellipsoid-shaped samples of skin from a patient. The skin cutter comprises a central hollow tube, a spring and rod combination within the tube. Also, it comprises gripping means combined with the skin cutting means comprising an assembly with multiple pivotable joints, and two skin cutting blades. The assembly carries out a scissoring motion when the rod is pressed down, causing the blades to come together and thereby remove the skin sample. The blades can have either concave cutting surfaces, or straight cutting surfaces that are deformed into an arc shape. Either blade cutting surface configuration allows for excision of the desired ellipsoid-shaped tissue sample from the patient. As the skin cutting blades cut through the skin in an angle at least partly parallel to the skin surface, it is not possible to cut deep without removing too much surface skin and there by delay healing of the wound and risk forming large scars.

Hence, an improved biopsy tool would be advantageous, and in particular a single biopsy tool comprising both a cutting means adapted to cut a specific biopsy size and position-wise, and a gripping means adapted to catch and hold the cut biopsy.

### Summary of the invention

Thus, an object of the present invention relates to providing a cheaper, more sustainable and more efficient skin punch biopsy tool for providing a sample from an outer layer of a human or animal body i.e. a sample from skin or mucous membrane.

Thus, one aspect of the invention relates to a skin punch biopsy tool for providing a skin or mucous membrane sample (15) comprising primary cutting means in form of a cutting edge (1) and gripping means (2, 2a, 2b) for gripping a skin sample (15), wherein the cutting edge(s) (1) is not part of the gripping means (2, 2a, 2b).

The primary cutting edge(s) 1 is/are separate from the gripping means and normally positioned at a different position than the gripping means 2, 2a, 2b and constituted of parts being completely or at least partly separated from the gripping means 2 i.e. the primary cutting means 1 are not constituted of the same part(s) as the gripping means 2. When the cutting means 1 are not part of the gripping means 2, 2a, 2b, it is possible to optimize both functions, i.e. it is possible to prepare a skin sample of size and shape as prescribed by a doctor or pathologist and it is possible to handle the skin sample with the gripping means as prescribed by a doctor or pathologist.

According to any embodiment of the biopsy tool, the cutting edge(s) (1) may be positioned at a first position or constitute(s) a first end of the biopsy tool, while the gripping means (2, 2a, 2b) are positioned at a second position, optionally at an opposite second end of the biopsy tool. Both the cutting edge (1) and the gripping means (2) may be secured to a central body which central body also may constitute a handle (3) or part of a handle for either the cutting edge (1) and/or for the gripping means (2). The handle part (3) may constitute a hold or a grip during cutting or punching and may also constitute a support for the operators' hand during operation of the gripping means (2).

According to any embodiment of the biopsy tool, the gripping means (2, 2a, 2b) may comprise two or more opposed flexible or flexibly mounted arms (2a, 2b) e.g. similar to a pair of tweezers. That the arms are "opposed" means that the arms fully or partly may be moved towards each other when operated.

According to any embodiment of the biopsy tool, the biopsy tool may comprise a punch part (4) comprising the primary cutting edge (1), which punch part (4) may comprise a longitudinally extending tube-like body having a round, ellipsoid or polyangular profile i.e. cross-section, forming a round, ellipsoid or polyangular cutting edge (1) positioned at an outer or forward end of the punch part (4).

According to any embodiment of the biopsy tool, the punch part (4) may be made of a single piece of material such as stainless steel or similar material being hard enough to constitute a cutting edge (1). The punch part (4) may be attached to or secured to the skin punch biopsy tool by means known to a skilled person such as during moulding, or the punch part (4) may be pressed into the skin punch biopsy tool e.g. during heating, or the punch part (4) may be attached to the skin punch biopsy tool by gluing or welding.

According to any embodiment of the biopsy tool, the biopsy tool may comprise a safety cap (6) configured to be at two positions, a first position where the safety cap (6) is at a retracted position relative to the cutting edge (1) where the safety cap (6) does not cover the cutting edge (1), and a second position where the safety cap (6) is at a forward position relative to the cutting edge (1) where the safety cap (6) covers the cutting edge (1) and prevents contact with the cutting edge (1). Optionally, the safety cap (6) may be locked in the forward position in such a way that the safety cap (6) cannot be brought back to the retracted position without using excessive force or activation of unlocking means.

According to any embodiment of the biopsy tool, the biopsy tool may comprise a handle part (3) configured to hold when moving the biopsy tool in an insertion direction defining a profile and the length of a skin sample (15). Optionally, the handle part (3) may constitute a central part of the biopsy tool and may support handling of the cutting means and/or the gripping means.

According to any embodiment of the biopsy tool, an outer end of an outer portion (12) of one or each arm (2a, 2b) may comprise a secondary cutting edge (7) and two opposing secondary cutting edges (7) may be configured to meet when the arms (2a, 2b) are forced together in a cutting direction thereby cutting and releasing the skin sample (15). Each cutting edge (7) may be straight and the minimum length of the cutting edge (7) may be at least 10%, or at least 20%, or at least 30% or at least 40%, or at least 50% or at least 60% or at least 65% or may be around 70% of the maximum cross-sectional dimension of the skin sample (15), and/or each cutting edge (7) may be smaller than 100% or smaller than 90% or smaller than 80% or may be smaller than 75% of the maximum cross-sectional dimension of the skin sample (15).

According to an embodiment of the biopsy tool, a part at the outer end portion (12) of one or each arm (2a, 2b) may comprise a recess or depression (8) at the inner surface (17) which recess or depression (8) may be either rounded as a spoon or comprise a surface with angularly positioned walls. When the two arms (2a, 2b) are forced together, the one or two recesses or depressions together may form a hollow configured to hold a cut-out skin sample.

According to an embodiment of the biopsy tool, two opposite arms (2a, 2b) of the gripping means (2) may mirror each other and may comprise an inclining outer surface (16) and an inclining inner surface (17) which inner and/or outer surfaces are inclined relative to the longitudinal direction defining an insertion direction. The inner surface s(17) of an arm 2a, 2b may be angled relative to the insertion direction by an angle α, where α > 5 degrees and α < 30 degrees, preferably α > 6 degrees and α < 20 degrees, preferably α > 7 degrees and α < 15 degrees, preferably α > 8 degrees and α < 11 degrees.

According to an embodiment of the biopsy tool, the outer end portion (12) of the gripping means (2, 2a, 2b) and an optional cutting edge (7) of the gripping means are adapted to the size and/or perimeter of an insertion gap (15a) formed by the cutting edge (1) and surrounding the skin sample (15) such that outer end portion (12) of the gripping means fit around the skin sample when inserted into the insertion gap (15a).

According to an embodiment of the biopsy tool, the biopsy tool may comprise a plunger (10) comprising a front surface (11) facing forward in an insertion direction, which plunger (10) is configured to be in two positions, a first position where the plunger (10) is at a retracted position relative to the cutting edge (1) allowing for cutting with the cutting edge (1), and a second position where the plunger (10) is at a forward position where the front surface (11) of the plunger extends to or beyond cutting edge (1).

According to an embodiment of the biopsy tool, the biopsy tool may comprise both a safety cap (6) and a plunger (10) which safety cap (6) and plunger (10) are mechanically connected and move together with same velocity and in same direction.

According to an embodiment of the biopsy tool, the biopsy tool may be either disposable or partly disposable or reusable.

According to an embodiment of the biopsy tool, a handle part (3), the gripping means (2) and an extending part configured to hold or for mounting of a punch part (4) may be manufactured in one piece via a manufacturing technique such as moulding e.g. injection moulding, or compression moulding, or blow moulding, or by thermoforming, or rotational moulding or by 3D-printing, CNC Machining or Extrusion and the punch part (4) comprising the cutting edge (1) is during manufacturing fixed to the extending part.

### Brief description of the figures

Figure 1 shows an embodiment of a biopsy tool according to the invention.
Figures 2A and 2B shows one end of an embodiment of a biopsy tool according to the invention comprising a safety cap in respectively a retracted state and a forward state.
Figure 3 shows the outer end and part of gripping means according to the invention in a view showing a recess of holding a biopsy.
Figure 4 shows the outer end and part of gripping means according to the invention in a side-view showing the angular of the arms of the gripping means.
Figures 5A-5C illustrates angular positions of two opposing arms functioning as gripping means of an embodiment of a biopsy tool.
Figures 6A-6B illustrates an embodiment of a biopsy tool comprising a plunger.

### Detailed description of the invention

### Definitions

Prior to discussing the present invention in further details, the following terms and conventions will first be defined:
*In general* - when this expression is used when mentioning a feature relating to the present invention, it must be understood that the feature may be used with all embodiments of the invention, even if the mentioning is made in the detailed part of the document.
*Oppose* - means that two or more parts are placed opposite or against each other and may move towards each other.

The present invention will now be described in more detail in the following.

In general, a biopsy tool according to the invention comprises primary cutting means comprising a cutting edge 1 used to cut an insertion gap 15a in a direction of insertion. Normally, the cutting edge 1 comprises a continuous or closed cutting edge 1 e.g. having a round profile able to provide a sample having a longitudinal shape such as a cylindrical body. defining a cross-sectional profile of a skin sample.

In general, the cutting edge 1 may be part of a punch part (4) made of a single piece of material such as stainless steel or similar material being hard enough to constitute the cutting edge 1, e.g. the punch part 4 may have a continuous or closed cutting edge 1 and a length lₚ being longer than the minimum length l of a desired cutting depth.

The biopsy tool also comprises gripping means 2, 2a, 2b for catching and removing the skin sample, and optionally the biopsy tool comprises means for releasing the skin sample at the lower end of the skin sample. Means for releasing a skin sample may comprise one or more secondary cutting edges 7.

A biopsy tool according to the invention is configured to provide a skin sample with a cut directed approximately perpendicular to the skin surface, traditionally known as a punch biopsy. A cut being approximately perpendicular to the skin surface makes it possible to prepare a relatively deep skin sample, e.g. being around 3-10 mm deep, while minimizing the upper/outer surface of the wound which after cutting is accessible to microorganisms and therefore infections.

The cut is made with the cutting edge 1, which normally comprises a continues or closed cutting edge 1 has e.g. a round profile able to provide a sample having a longitudinal shape such as a cylindrical body. The cutting edge 1 may constitute a flat profile i.e. a profile being approximately either parallel to the skin surface upon cutting or inclined relative to the skin surface upon cutting. Alternatively, the cutting edge 1 may be curved, e.g. the cutting edge 1 may comprise one or more concave and/or convex sections e.g. providing a wavelike profile.

When using the primary cutting means for cutting, the cutting edge 1 can either be rotated while being pushed against the skin surface during cutting, or the cutting edge 1 can be forced or punched into the skin surface in a direction perpendicular to the skin surface without turning the cutting edge 1. If the cutting edge 1 is rotated as well as pushed against the skin surface during cutting, the cutting edge 1 normally has a round profile to reduce damage to the skin surface.

One advantage of a biopsy tool according to the invention is that one biopsy tool according to the invention may replace either two or three traditional tools for preparing a biopsy sample.

According to an embodiment, a biopsy tool may replace a primary cutting means, and a gripping means similar e.g. to a set of tweezers. Such an embodiment may comprise gripping means comprising two or more opposing arms 2a, 2b extending in a longitudinal direction approximately parallel to each other where each arm 2a, 2b has an outer end 12 to be inserted into an insertion gap during use, and an inner end 13 fixed to a handle part of the biopsy tool. When the gripping means are used to grab the skin sample and pull the skin sample out of the insertion gap, a scalpel or a scissor may be used to release the skin sample from the skin by cutting the lower end of the skin sample. If the skin sample is porous or disjointed it may not be necessary to cut the lower end of the skin sample as the skin sample may be released simply by pulling.

According to an embodiment, the biopsy tool may replace a primary cutting means, a gripping means such as a set of tweezers and a scalpel or scissors used to cut the skin sample free at the lower end of the sample. Such an embodiment may comprise gripping means comprising two or more opposing arms 2a, 2b extending in a longitudinal direction approximately parallel to each other where each arm 2a, 2b has an outer end 12 to be inserted into an insertion gap during use, and an inner end 13 fixed to a handle part of the biopsy tool. Also, the one, two or more opposing arms 2a, 2b may comprise a secondary cutting edge 7 at the outer end 12 of the gripping means and when the outer end 12 is inserted into the insertion gap defining the circumference of the skin sample, the at least one cutting edge 7 may be moved in a direction approximately perpendicular to the direction of insertion of the cutting edge 1.

According to any embodiment of the present invention, the primary cutting means and the gripping means are constituted of different parts and are positioned at different positions of a biopsy tool. Optionally, the primary cutting means and the gripping means may extend into different directions such as opposite directions from a common central part or common body part of the biopsy tool. The common central part or body part may constitute a handle 3 to be used when handling the primary cutting means of the biopsy tool.

In general, the two or more opposing arms 2a, 2b may be either flexibly mounted to the common central part or common body part of the biopsy tool, or they may be rigidly mounted but be constituted of a flexible material, in order to move either one or more of the outer ends 12 of the opposing arms 2a, 2b in a direction towards each other which direction may also be described as horizontal or perpendicular to the direction of insertion.

An advantage of positioning the parts constituting respectively the primary cutting means and the gripping means separately, is that it is possible to optimize the functionality of each of the parts creating two optimized tools. It is also possible to adapt the two tools to each other, e.g. the primary cutting means may cut in a direction, a prescribed size and a depth which is optimal for preparing a specific skin sample and the gripping means may on the other hand be optimized and adapted to slide into the insertion gap prepared by the primary cutting means of the same biopsy tool and grab the skin sample without crushing or otherwise destroying the sample.

In general, a biopsy tool according to the invention may be constructed as a reusable tool which may be used multiple times and is adapted to be subjected to a sterilization process e.g. at high temperature, by steam, by gas or another known sterilization process.

A reusable embodiment of a biopsy tool according to the invention may be prepared from a more expensive and durable material such as metal e.g. stainless steel, and the cutting edge 1 may constitute a build-in part of the structure.

Alternatively, a biopsy tool according to the invention may in general be constructed as a disposable tool or partly disposable tool, where a "disposable tool" is disposed of in its entirety after use, and a "partly disposable tool" comprises at least one part which may be used multiple times and may be adapted to be removed and to be sterilized.

A disposable or partly disposable embodiment of a biopsy tool according to the invention may be prepared from one, two or more parts, and one part may be prepared by moulding or printing of a polymeric material such as PP, ABS, PA, PET or similar. This part of polymeric material may be combined with a piece of a hard material such as a metal e.g. stainless steel, which is suitable for creating a sharp cutting edge constituting the primary cutting means comprising the cutting edge 1.

In general, the primary cutting means comprising the cutting edge 1 may comprise a pipe or tube-like body, e.g. made of metal such as stainless steel, extending in a longitudinal direction and having a constant profile in a cross-sectional direction. The cutting edge 1 is positioned at a first end or an outer end of the tube-like body whereas the opposite end of the tube-like body is fixed to a handle part 3 of the biopsy tool. The cutting edge 1 has the same profile as the constant cross-sectional profile of the tube-like body. The constant cross-sectional profile is traditionally round but may have any shape such as polygonal, or oval, as the shape of the cross-sectional profile may be adapted to a specific purpose.

In general, a biopsy tool according to the invention may be produced in a series of sizes and/or cross-sectional profiles making it possible for a healthcare professional such as a doctor or nurse or veterinarian to choose a biopsy tool suitable for a given task.

In general, a skin punch biopsy tools may be provided as a series of tools having different sizes of primary cutting means 1 where the cutting edge of each tool has a cross-section or diameter of between 1-14 mm, and a depth - or length of tube-like body - of between 3 and 10 mm. A series therefore may comprise a number of skin punch biopsy tools each comprising a primary cutting means 1 of a fixed size, when an operator wants to take a skin biopsy of a given size, the operator chooses a skin punch biopsy tool having a primary cutting means 1 of the desired size. According to a preferred embodiment, the primary cutting means 1 and the gripping means 2 of each skin punch biopsy tool belonging to a series are adapted to each other and the gripping means 2 may comprise two or more opposing parts extending in a direction parallel to each other or at sections inclined relative to each other in such a way that the opposing gripping means 2 are easily inserted into the insertion gap 15a provided by the cutting edge 1 of the same biopsy tool, while a portion of the outer end 12 of the gripping means also grab gently around the skin sample.

When the gripping means comprise two or more opposing parts each comprising an outer end portion 12 to be inserted into the insertion gap, then the outer end portion 12 of one or more opposing parts may comprise a cutting edge 7. Such a cutting edge 7 points or cuts in a horizontal direction which horizontal direction is essentially perpendicular to the direction of insertion. Each cutting edge 7 may comprise a straight or triangular or rounded cutting edge 7 depending on whether one, two, three or more opposing arms 2a, 2b comprise(s) a cutting edge 7, and whether this or these one, two, three or more cutting edge(s) 7 meet one or more cutting edge(s) 7 at the outer end 12 when the opposing arms are being squeezed together.

According to a preferred embodiment, the two or more opposing gripping means are each adapted in size and in shape to fit into the insertion gab made of the corresponding cutting means of the same biopsy tool. If the insertion gap is round, and the gripping means comprise two opposing arms 2a, 2b, then the width of the cutting edge 7 of one arm of the gripping means may be straight and the width of the cutting edge 7 may be 40-90%, or 50-80%, or 60-80%, or around 70% of the diameter of the cutting edge 1 corresponding to the diameter of the insertion gap. The outer end portion 12 of each arm of the gripping means may be rounded in cross-section direction to fit the circular formed by the insertion gap when in use, the cross-sectional direction being perpendicular to the insertion direction in a use-situation.

In general, an outer portion of one or more of two or more opposing arms of the gripping means, may comprise a spoon-like recess 8 which prevents squeezing of the skin sample. It also has a 'spoon' effect in the case of porose tissue where the tissue otherwise easily falls apart, when gripping it.

The "outer portion" 12 of each arm of the gripping means 2 may constitute 10-30 % of the free length of an opposing arm 2a, 2b measured from the outer end e.g. defined by the cutting edge 7. The outer portion 12 may be adapted to the length or depth of the skin sample, e.g. around 10 mm, of one or of each arm 2a, 2b.

In general, a biopsy tool according to the invention may comprise a plunger 10 comprising a front surface 11, which plunger 10 is adapted to pass from a retracted position inside the punch part 4 to a forward position. At the retracted position, the front surface 11 of the plunger 10 defines a closed end or partly closed end of the primary cutting means and in the forwards position the front surface 11 of the plunger 10 is at level with the cutting edge 1 or has passed beyond the cutting edge 1. During use of the biopsy tool, the plunger 10 may push a cut sample out of a tube-like body which - completely or partly - constitutes the primary cutting means. The plunger 10 may after use provide a protection against accidentally getting into contact with the sharp cutting edge 1, especially if the front surface 11 of the plunger extends beyond the cutting edge 1 in the forward position.

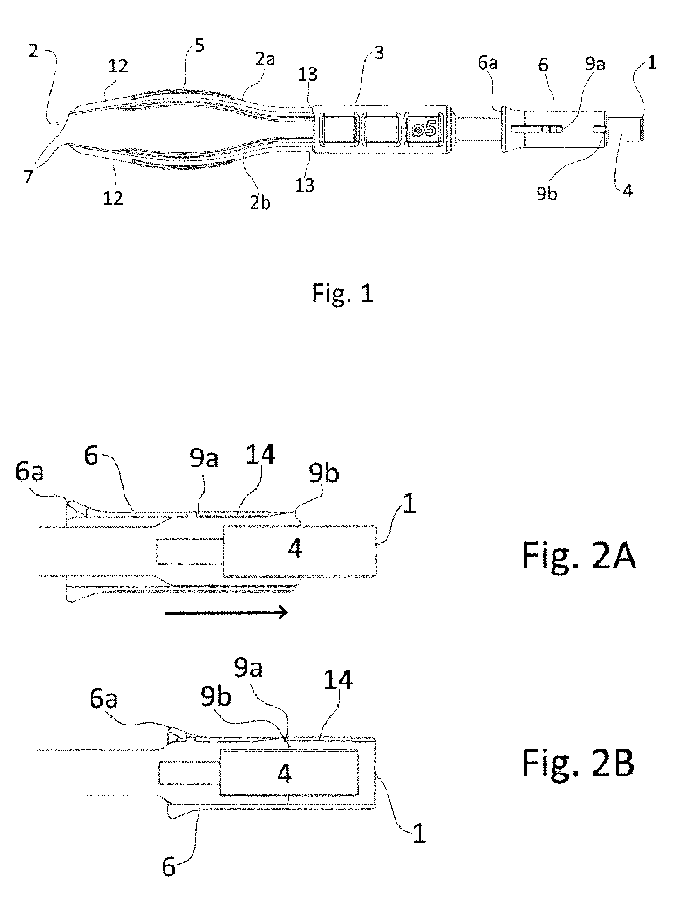

Figure 1 shows an embodiment of a biopsy tool according to the invention. The shown embodiment is a disposable tool which may be moulded or printed of a polymer material. This embodiment a comprises a cutting edge 1 being part of a punch part 4, the punch part 4 is attached to a handle part 3 making it possible to manually forcing the cutting edge 1 into the skin to obtain a skin sample. According to this embodiment, the handle part 3 constitutes a central part of the tool and the handle part 3 may also comprises printed information about the tool such as size of the cutting edge 1 e.g. 05mm which makes it easier for the user to identify the size after the tool is taken out of the sterile packing. Normally, the punch part 4 is a hollow tube-like part where the cutting edge 1 constitutes the front surface or front profile of the punch part 4. When the punch part 4 is moved forward in direction of the cutting edge 1, the cutting edge 1 will cut a round or otherwise profiled insertion gap defining a skin sample having a free upper end and a lower end which is still attached to the body from where the skin sample is to be removed. The shown embodiment belongs to a series of tool and as printed on the handle 3 of the embodiment, the specific embodiment of the tool has a punch part 4 comprising primary cutting means 1 having a diameter of 5 mm.

The shown embodiment comprises a safety cap 6. The safety cap 6 and the part of the primary cutting means to which the punch part 4 is attached comprise corresponding locking means 9a, 9b in form of an opening and contact surface of the safety cap 6 and a protruding part 9b of the handle/punch part 3, 4. The safety cap 6 comprises a push surface 6a which makes it possible for a user to push the safety cap 6 to a forward and optionally locked position where the safety cap 6 covers the cutting edge 1.

The shown embodiment also comprises gripping means 2 constituted of two longitudinally extending arms 2a and 2b having an inner end 13 being mounted to the handle part 3 and an opposite outer end portion 12 where the two arms 2a, 2b in a relaxed state is positioned at a distance to each other. The flexibility of the two arms 2a, 2b or a flexible mounting of at the inner end 13 allows the two outer ends of the arms 2a, 2b to meet when pushed together. One or each of the two arms 2a, 2b may both comprises a cutting edge 7 at the outer end making it possible to cut a skin sample free at the lower end before pulling the skin sample up and out of the surrounding tissue.

Each of the arms 2a, 2b may comprise a handle part 5 which may provide a rough or non-slippery surface where the user may obtain a good and controllable grip even with wet hands or hands covered by latex or rubber gloves.

Figures 2A and 2B shows one end of an embodiment of a biopsy tool, a punching or cutting end, comprising a safety cap in respectively a retracted state and a forward state.

Fig. 2A illustrates the retracted state of the safety cap 6. In general, a safety cap 6 may be locked in the retracted state by locking means 9 which locking means 9 may comprise corresponding surfaces 9a, 9b which prevent movement of the safety cap 6 in one direction. According to the shown embodiment, the safety cap 6 comprises a tube-shaped member with a longitudinal side opening 14, the locking means 9 comprises in the shown embodiment a surface 9a of the side-opening 14 of the safety cap and a protruding surface 9b protruding from a surface of the primary cutting means. An inclined surface of the protruding part comprising the surface 9b (facing left in figs. 2A and 2B) allows for a part of the tube-shaped safety cap 6 to slide over the protruding part comprising the surface 9b when subjected to force applied to the push surface 6a in the direction indicated with the arrow below the embodiment of fig. 2A. The surface 9a of the safety cap 6 facing opposite the direction of the arrow and defining an end of a side-opening 14 in the safety cap 6 provide the locking means 9 of the safety cap 6. In the forward position of the safety cap 6 shown in fig. 2B, the surface 9a of the opening of the safety cap 6 faces the surface 9b of the protruding part of the primary cutting means. As the surface 9b of the protruding part is steep, the safety cap 6 cannot pass back to the retracted position and the safety cap 6 is locked in the forward position. The shown locking means for the safety cap are very simple and there may be other ways for a skilled person to construct such locking means with corresponding surfaces.

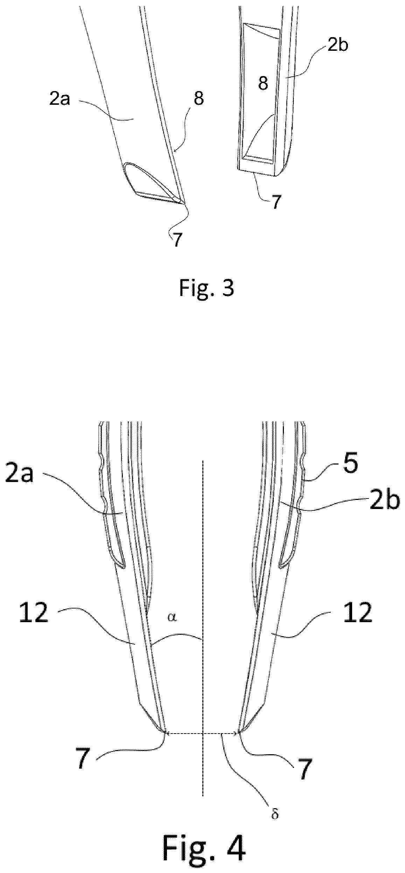

Figure 3 shows an enlargement of an outer end portion 12 of gripping means comprising two opposing arms 2a, 2b according to the invention in a view showing a recess 8 for receiving a skin sample during use. This view of the gripping means 2a, 2b illustrates how a recess 8 may be provided in each arm 2a, 2b of the gripping means 2. An inclining outer surface (i.e. the lower surface closest to the cutting edge 7 as illustrated in fig. 3) of each arm 2a, 2b makes it possible to insert the gripping means into the insertion gap made by the punch part 4 of the primary cutting means positioned at a different position. In general, the shape and size of the gripping means 2, is adapted to the punching part 4 in such a way that the outer end portion 12 of the gripping means fits into the cut defined by the size of the cutting edge 1.

Figure 4 shows a side-view of an embodiment according to the invention of gripping means 2 comprising two opposing arms 2a, 2b each provided with an outer end portion 12. The view shows how the outer portions 12 of each of the opposing arms 2a, 2b in a relaxed position are positioned in a distance δ to each other at the outer end. In general, the distance δ between two opposing arms in a relaxed state may be adapted to primary cutting edge 1 or punch part 4 which is also constituting a part of skin biopsy tool according to the invention. In the shown embodiment, the outer end of each arm 2a, 2b comprises a cutting edge 7. Also, the outer portion 12 of the arms 2a, 2b are positioned at an angle α relative to the insertion direction or the longitudinal direction of the device. The angle α relative to the insertion direction, or the angle 2 x α defining the angle between the inner surfaces 17 of the outer portions 12 of the arms 2a, 2b, may support pushing tissue aside which surrounds a cut-out skin sample when the gripping means 2 are inserted into a circular or otherwise shaped insertion gap 15a made by a punch part 4. Pushing the surrounding tissue aside facilitates releasing of the skin sample when forcing the arms 2a, 2b together and e.g. cutting the skin sample free with a cutting edge 7 of the gripping means 2 at the lowest position of the skin sample 15. This is done without squeezing and pulling the volume of the skin sample, or at least with less squeezing and pulling of the volume of the skin sample than done with traditional gripping means, and this reduces the risk of damaging the tissue sample.

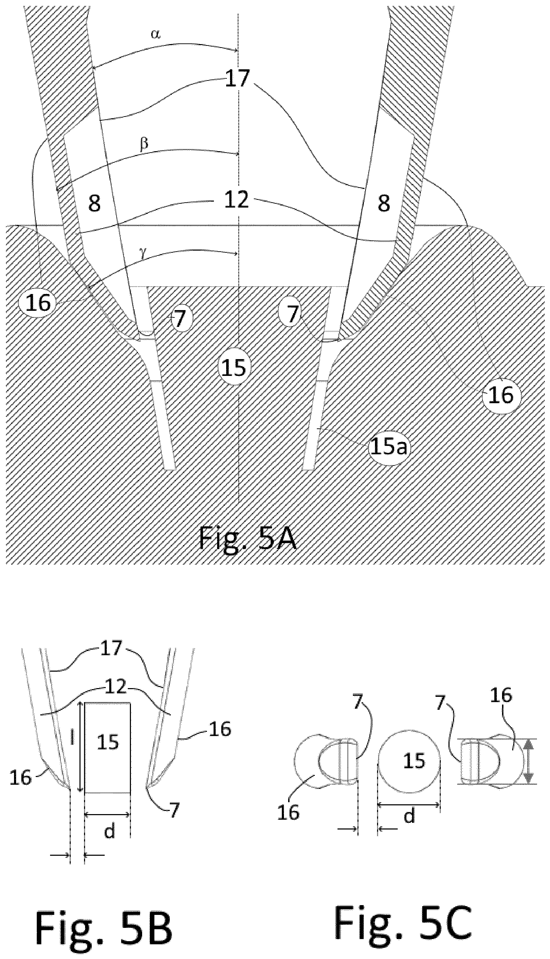

Figures 5A-5D illustrate a side-view of a skin sample 15 which in a cross-sectional or horizontal direction is defined by an insertion gap 15a. The insertion gap 15a is prepared or formed by cutting in the insertion direction with the primary cutting means may be positioned and dimensioned relative to the gripping means 2. The insertion direction may be perpendicular to the skin surface, or the insertion direction may be angled relative to the skin surface, the choice of insertion direction is based on the knowledge of the person taking the skin sample.

Fig. 5A illustrates what happens when inserting the gripping means 2a, 2b into the insertion gap 15a, when the gripping means 2a, 2b are fully inserted the skin sample may be released by forcing the opposing arms 2a, 2b of the gripping means together.

In general, when there are only two opposing arms 2a, 2b, the arms preferably mirror each other.

In general, when there are only two opposing arms 2a, 2b, each arms 2a, 2b has an inner surface 17 facing the oppositely position arm, and each arms 2a, 2b has an outer surface 16 facing away from the oppositely position arm. According to a preferred embodiment, the inner surface 17 of the outer portion 12 of each arm 2a, 2b is inclined relative to the insertion direction at an angle α where 5° < α < 15°, or 8° < α < 11°. According to a preferred embodiment, the outer surface 16 of the outer portion of each arm 2a, 2b is inclined relative to the insertion direction at an angle of at least the same size as the angle α, preferably the inclination angle of the outer surface 16 increases at the outer portion 12, normally close to the outer end, from α to at least 45°, or at least 60°, or at least 80°, and may preferably meet the inner surface 17 at or near a secondary cutting edge 17.

Fig. 5A illustrates how the inclination of the outer surface 16 may be changed in steps, and at a first step - furthest away from the outer end of the arm - the outer surface 16 is parallel to the inner surface and has an angle α relative to the insertion direction in a relaxed state. In a second step the outer surface is inclined relative to the inner surface 17 and has an angle β relative to the insertion direction in a relaxed state. In a third step, the outer surface 16 is further inclined relative to the inner surface 17 and has an angle γ relative to the insertion direction in a relaxed state. In a fourth and last step, the outer surface 16 is rounded towards or otherwise approaching the inner surface 17 defining the outer end of the arm 2a, 2b.

In general, the angle α < the angle β < the angle γ and in the last step the angle of the outer surface is further increasing and may approach 90°. In the embodiment shown in fig. 5A, the angle α is 10°, the angle β is 35°, and the angle γ is 65°.

When one or each arm 2a, 2b comprises a straight or slightly curved cutting edge 7, it has shown to be advantageous to provide one or each arm 2a, 2b with an inclined outer surface 16 in the longitudinal direction or the insertion direction which provides a slightly increasing outer diameter or other cross-sectional profile near the upper or outer end of the skin sample as the gripping means 2 are inserted into the insertion gap 15a.

According to the embodiment shown in fig. 5A the gripping means 2 comprising the two opposing arms 2a, 2b are in a relaxed state and have been inserted into the outer or upper part of the insertion gap 15a. The skin surrounding the insertion gap 15a is pushed away from the insertion gap 15a forming a bulge which is in contact with the outer surface 16 of the arms 2a, 2b.

When having reached as low as desired into the insertion gap 15a to obtain a desired volume of skin sample 15, the gripping means 2a, 2b are pushed or pressed together and the straight cutting edges 7 of the gripping means then cut and release the skin sample 15 by cutting through at least a part of the cross-section of the skin sample. The recess 8 in both the opposing arms 2a, 2b protects especially soft or loose skin samples 15 from being squeezed or otherwise damaged during the removal process.

Figures 5B and 5C illustrate in respectively a side view and an end view, dimensions of the gripping means 2 comprising two opposing arms relative to the skin sample 15 and positioning of the gripping means 2 during catching and releasing of the skin sample 15.

Fig. 5B shows a side view of a skin sample 15 and how the gripping means 2 comprising two arms 2a, 2b having outer portions 12 are dimensioned relative to the skin sample 15. Normally, a skin sample 15 has a diameter or cross-section dimension d between 1 - 10 mm, and a length or depth l of between 3-10 mm.

The outer portions 12 of the arms 2a, 2b correspond in cross-sectional size and shape to the insertion gap 15a made by the cutting edge 1 of the punch part of the biopsy tool according to the invention.

In a cross-sectional view, the shape of the arms 2a, 2b are adapted to the shape of the insertion gap 15a made by the cutting edge 1. In the shown example of fig. 5B and 5C, the skin sample 15 has a circular cross section, the insertion gap 15a is therefore round and in order for the arms 2a, 2b to fit into the insertion gap 15, the outer surface 16 is shaped as part of a circular arch as may be seen in fig. 5C.

In general, the outer surface 16 of each arm 2a, 2b may be curved in a cross-sectional or horizontal direction and corresponding to the shape and angle as the insertion gap 15a. A cross-sectional or horizontal direction is perpendicular to the insertion direction.

Also, if the skin sample 15 has a round cross-section, the width of a straight cutting edge 7 of an arm 2a, 2b may constitute less than 100% of the maximum cross-sectional width such as less than 100% of the diameter of the skin sample 15. Preferably less than 90%, or less than 80% of the maximum cross-sectional width of the skin sample, and/or the width of the cutting edge 7 of each arm may constitute at least 40% of the maximum diameter, or at least 50% of the maximum of the diameter of the skin sample 15.

### Example 1

The desired skin sample must have a diameter of 3 mm.

The skin punch biopsy tool used for providing the skin sample comprises a punch part 4 comprising cutting edge 1 constituted of a round/cylindric metal unit having a diameter of 3 mm and a free length of 8 mm.

The gripping means 2 comprises two opposing arms 2a, 2b each comprising a straight secondary cutting edge 7. The length/width of each cutting edge 7 constitutes 70% of 3 mm i.e. 2.1 mm.

In a cross-sectional view, the outer surface 16 of the outer portion 12 of each arm 2a, 2b is curved as a circular arch reaching from one end of the straight cutting edge 7 to the other end of the cutting edge 7.

In a longitudinal view i.e. in a view parallel to the insertion direction, the outer surface 16 of the outer portion of each arm 2a, 2b, is step-wise approaching the angle of the inner surface 17. In a first section the angle α is 9°, in the second section the angle β is 35°, and in the third section the angle γ is 65°, in the last section closest to the cutting edge 7, the angle is 80°. In a relaxed state, the distance δ between the arms 2a, 2b is 2 mm larger than the diameter of the skin sample 15, i.e. 5 mm.

### Example 2

The desired skin sample must have a diameter of 8 mm.

The skin punch biopsy tool used for providing the skin sample comprises a punch part 4 comprising cutting edge 1 constituted of a round/cylindric metal unit having a diameter of 8 mm and a free length of 8 mm.

The gripping means 2 comprises two opposing arms 2a, 2b each comprising a straight secondary cutting edge 7. The length/width of each cutting edge 7 constitutes 70% of 8 mm i.e. 5.6 mm.

In a cross-sectional view, the outer surface 16 of the outer portion 12 of each arm 2a, 2b is curved as a circular arch reaching from one end of the straight cutting edge 7 to the other end of the cutting edge 7.

In a longitudinal view i.e. in a view parallel to the insertion direction, the outer surface 16 of the outer portion of each arm 2a, 2b, is step-wise approaching the angle of the inner surface 17. In a first section the angle α is 9°, in the second section the angle β is 35°, and in the third section the angle γ is 65°, in the last section closest to the cutting edge 7, the angle is 80°. In a relaxed state, the distance δ between the arms 2a, 2b is 2.2 mm larger than the diameter of the skin sample 15, i.e. 10.2 mm.

"Free length" means that the cutting edge 1 may be inserted into a depth of the skin defined by the free length.

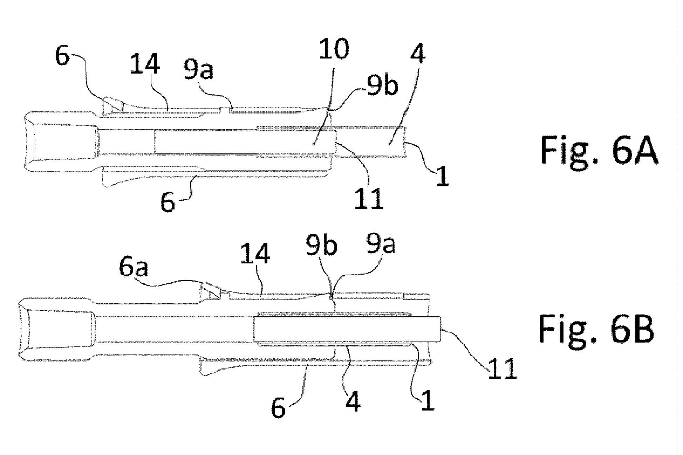

Figure 6A and 6B show an embodiment of a primary cutting means of a biopsy tool according to the invention comprising both a safety cap 6 and a plunger 10 comprising a front surface 11.

Fig. 6A shows the safety cap 6 and the plunger 10 in a retracted position whereas fig. 6B shows the safety cap 6 and the plunger in a forward position. According to this embodiment, the safety cap 6 and the plunger 10 are moved from the retracted to the forward position simultaneously as they are mechanically connected, however, this is not a requirement for the invention as such, i.e. the safety cap 6 and the plunger 10 may move independently of each other.

If the safety cap 6 and the plunger 10 are not mechanically connected, then the plunger 10 comprises activation means which may be activated and push the plunger to the forward position, if the skin sample is stuck inside the punch part 4 after the skin sample 15 has been released from the skin.

| **Ref. no.** | **Ref. name** |
|---|---|
| 1 | Cutting edge, primary cutting edge |
| 2, 2a, 2b | Gripping means, opposite or opposing arms |
| 3 | Handle part for cutting means |
| 4 | Punch part |
| 5 | Handle part for gripping means |
| 6 | Safety cap |
| 6a | Push surface for safety cap |
| 7 | Cutting edge of gripping means, secondary cutting edge |
| 8 | Recess or depression position at the outer part of an arm 2a or 2b |
| 9 | Locking means for safety cap |
| 9a, 9b | Corresponding surfaces providing locking means for safety cap |
| 10 | Plunger |
| 11 | Front surface of plunger |
| 12 | Outer end portion of opposing arms |
| 13 | Inner end of opposing arms |
| 14 | Longitudinal side opening of safety cap |
| 15 | Skin sample |
| 15a | Insertion gap defining depth and cross-sectional profile of skin sample |
| 16 | Outer surface of gripping means |
| 17 | Inner surface of gripping means |

## Claims

1. A skin punch biopsy tool for providing a skin or mucous membrane sample (15) comprising a cutting edge (1) and gripping means (2, 2a, 2b) for gripping a biopsy sample, **characterized in that** the cutting edge(s) (1) is not part of the gripping means (2, 2a, 2b).

2. A skin punch biopsy tool according to claim 1, wherein the cutting edge(s) (1) is/are positioned at a first position or constitute(s) a first end of the biopsy tool, and the gripping means (2, 2a, 2b) are positioned at a second position, preferably at an opposite second end of the biopsy tool.

3. A skin punch biopsy tool according to any previous claim, wherein the gripping means (2, 2a, 2b) comprises two or more opposed flexible or flexibly mounted arms (2a, 2b) e.g. corresponding to a pair of tweezers.

4. A skin punch biopsy tool according to any previous claim, wherein the biopsy tool comprises a punch part (4) comprising the cutting edge (1) which punch part (4) comprises a longitudinally extending tube-like body having a round, ellipsoid or polyangular profile forming a round, ellipsoid or polyangular cutting edge (1) positioned at an outer end of the punch part (4).

5. A skin punch biopsy tool according to any previous claim, wherein the biopsy tool comprises a safety cap (6) which may be positioned at two positions, a first position where the safety cap (6) is at a retracted position relative to the cutting edge (1) where the safety cap (6) does not cover the cutting edge (1), and a second position where the safety cap (6) is at a forward position relative to the cutting edge (1) covering the cutting edge (1) and preventing contact with the cutting edge (1).

6. A skin punch biopsy tool according to any previous claim, wherein the biopsy tool comprises a handle part (3) at least configured to hold when moving the biopsy tool in an insertion direction defining a profile and the length of a skin sample (15).

7. A skin punch biopsy tool according to claim 3, wherein an outer end of an outer portion (12) of each arm (2a, 2b) comprises a cutting edge (7) and the two cutting edges (7) are configured to meet when the arms (2a, 2b) are forced together in a cutting direction thereby cutting and releasing the skin sample (15).

8. A skin punch biopsy tool according to claim 7, wherein the cutting edges (7) are straight and the minimum length of the cutting edge (7) is at least 10%, or at least 20%, or at least 30% or at least 40%, or at least 50% or at least 60% or at least 65% or is around 70% of the maximum cross-sectional dimension of the skin sample (15), and/or is smaller than 100% of the maximum cross-sectional dimension of the skin sample (15), and/or is smaller than 90% of the maximum cross-sectional dimension of the skin sample (15), and/or is smaller than 80% of the maximum cross-sectional dimension of the skin sample (15), and/or is smaller than 75% of the maximum cross-sectional dimension of the skin sample (15).

9. A skin punch biopsy tool according to claim 3 or claim 8, wherein a part at the outer end portion (12) of one or of each arm (2a, 2b) comprises a recess or depression (8) at the inner surface (17) which recess or depression (8) may be either rounded as a spoon or comprise a surface with angularly positioned walls.

10. A skin punch biopsy tool according to claim 3, wherein two opposite arms (2a, 2b) of the gripping means (2) mirror each other, and may comprise an inclining outer surface (16) and an inclining inner surface (17) which surfaces (16, 17) are inclined relative to the longitudinal direction defining an insertion direction.

11. A skin punch biopsy tool according to any previous claim, wherein an outer end portion (12) of the gripping means (2, 2a, 2b) and an optional cutting edge (7) of the gripping means are adapted to the size and/or perimeter of an insertion gap (15a) formed by the cutting edge (1) and surrounding the skin sample (15) such that outer end portion (12) of the gripping means fit around the skin sample when inserted into the insertion gap (15a).

12. A skin punch biopsy tool according to any previous claim, wherein the biopsy tool comprises a plunger (10) comprising a front surface (11) facing forward in an insertion direction, which plunger (10) is configured to be in at least two positions, a first position where the plunger (10) is at a retracted position relative to the cutting edge (1) allowing for cutting with the cutting edge (1), and a second position where the plunger (10) is at a forward position where the front surface (11) of the plunger extends to or beyond cutting edge (1).

13. A skin punch biopsy tool according to any previous claim, wherein the biopsy tool comprises both a safety cap (6) and a plunger (10) which safety cap (6) and plunger (10) may be mechanically connected and move together with same velocity and in same direction.

14. A skin punch biopsy tool according to any previous claim, wherein the biopsy tool is either disposable or partly disposable or reusable.

15. A skin punch biopsy tool according to any previous claim, wherein a handle part (3), the gripping means (2) and an extending part configured to hold or mount a punch part (4) are manufactured in one piece by moulding such as injection moulding, or compression moulding, or blow moulding, or by thermoforming, or rotational moulding or by 3D-printing, CNC Machining or Extrusion and the punch part (4) comprising the cutting edge (1) is during manufacturing fixed to the extending part.
